(19) Europäisches Patentamt
European Patent Office
Office européen des brevets

(11) EP 2 596 498 B1

(12) **FASCICULE DE BREVET EUROPEEN**

(45) Date de publication et mention de la délivrance du brevet:
**02.07.2014 Bulletin 2014/27**

(21) Numéro de dépôt: **11752594.9**

(22) Date de dépôt: **20.07.2011**

(51) Int Cl.:
***G11B 7/249*** *(2013.01)* ***B41M 5/26*** *(2006.01)*
***C07F 15/02*** *(2006.01)*

(86) Numéro de dépôt international:
**PCT/FR2011/051750**

(87) Numéro de publication internationale:
**WO 2012/010801 (26.01.2012 Gazette 2012/04)**

(54) **PROCEDE DE PHOTOCOMMUTATION THERMIQUE DE MATERIAUX A TRANSITION DE SPIN ET APPLICATIONS**

VERFAHREN FÜR DIE THERMISCHE PHOTOSCHALTUNG VON SPIN-ÜBERGANGSMATERIALIEN SOWIE IHRE VERWENDUNG

METHOD FOR THE THERMAL PHOTOSWITCHING OF SPIN-TRANSITION MATERIALS, AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.07.2010 FR 1055975**

(43) Date de publication de la demande:
**29.05.2013 Bulletin 2013/22**

(73) Titulaire: **Centre National de la Recherche Scientifique**
**75016 Paris (FR)**

(72) Inventeurs:
- **LETARD, Jean-François**
  **F-33610 Canejan (FR)**
- **FREYSZ, Eric**
  **F-33600 Pessac (FR)**

(74) Mandataire: **Goulard, Sophie et al**
**Ipsilon Brema-Loyer**
**Le Centralis**
**63, avenue du Général Leclerc**
**92340 Bourg-la-Reine (FR)**

(56) Documents cités:
**WO-A1-2007/065996 FR-A1- 2 755 696**
**FR-A1- 2 917 410**

- **GALLÃ CR G ET AL: "Room temperature study of the optical switching of a spin crossover compound inside its thermal hysteresis loop", APPLIED PHYSICS LETTERS, AIP, AMERICAN INSTITUTE OF PHYSICS, MELVILLE, NY, US, vol. 96, no. 4, 27 janvier 2010 (2010-01-27), pages 41907-41907, XP012131891, ISSN: 0003-6951, DOI: DOI:10.1063/1.3294312**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne un procédé de photocommutation thermique de composés à transition de spin et l'utilisation d'un tel procédé pour le marquage temporaire ou permanent de matériaux comprenant des particules d'au moins un composé à transition de spin.

Les composés impliquant l'ion fer(II) à base de ligand triazole, et les matériaux les incorporant, sont connus pour présenter le phénomène de transition de spin, associant un effet mémoire (hystérésis thermique autour de la température ambiante) avec une modification des propriétés optiques (changement de couleur), magnétiques et structurales. Ces propriétés sont mises à profit pour une utilisation de ses matériaux pour diverses applications, notamment pour le stockage d'information. De tels composés peuvent être des complexes de coordination contenant un ou plusieurs centres métalliques ayant une configuration $3d^4$, $3d^6$ ou $3d^7$, un ou plusieurs ligands azotés, et un ou plusieurs anions, tels que décrits par exemple dans les demandes de brevet EP-0 543 465, EP-0 666 561, EP-0 745 986, EP-0 842 988, la demande internationale WO2007/065996 et la demande de brevet FR 2 917 410.

A titre d'exemple, la demande de brevet EP-0 543 465 décrit des composés à transition de spin à base de fer et leur utilisation pour le stockage d'information. Certains de ces composés répondent à l'une des formules suivantes :

- $FeL_3(NO_3)_2$ dans laquelle L est un ligand du type 1,2,4-triazole ou 4-amino-1,2,4-triazole associé à l'anion $NO_3^-$;
- $Fe(ATP)_2$,$5Cl_2$, dans laquelle le ligand ATP est le 4-amino-1,2,4-triazole associé à Fe(II) et à Cl- ;
- $Fe(TP)_2Cl_2$ dans laquelle le ligand TP est le 1,2,4-triazole, associé à $Cl^-$ ;
- [Fe(2-aminométhylpyridine)$_3$]$Cl_2$EtOH, EtOH étant l'éthanol ;
- [Fe(1,10-phénanthroline)$_2$](NCS)$_2$ ;
- [Fe(1-propyltétrazole)$_6$]($BF_4$)$_2$.

A l'exception du [Fe(1,10-phénanthroline)$_2$(NCS)$_2$], ces composés sont roses dans l'état bas spin (BS), et blancs dans l'état haut spin (HS).

La demande internationale WO2007/065996 décrit un matériau sous forme de nanoparticules de complexes répondant à la formule (I) suivante :

$$\left[\left(Fe_{1-y}M_yL_3\right)_w L_3\right]\left[X'_{2/x\left(1-z/x'\right)}Y'_{2z/x'}\right]_w \quad (I)$$

dans laquelle :

- L représente un ligand 1,2,4-triazole portant un substituant R sur l'azote en position 4 ;
- X' est un anion ayant la valence x, $1 \leq x \leq 2$ ;
- Y' est un anion différent de X ayant la valence x', $1 \leq x' \leq 2$ ;
- R est un groupe alkyle ou un groupe $R^1R^2N$- dans lequel $R^1$ et $R^2$ représentent chacun indépendamment de l'autre H ou un radical alkyle ;
- M est un métal ayant une configuration $3d^4$, $3d^5$, $3d^6$ ou $3d^7$, autre que Fe ;
- $0 \leq y \leq 1$, $0 \leq z \leq 2$, et $3 \leq w \leq 1500$.

Les composés décrits dans ce document sont roses dans l'état BS, et blancs dans l'état HS.

La demande de brevet FR 2 917 410 décrit un matériau à transition de spin constitué par au moins un composé répondant à la formule (II) suivante :

$AX_bY_c$ (II)

dans laquelle :

- A répond à la formule $Fe_{1-m}M_m(R\text{-}Trz)_3$ ;
- M est un métal ayant une configuration $3d^4$, $3d^5$, $3d^6$ ou $3d^7$, autre que Fe ;
- $0 \leq m \leq 1$ ;
- R-Trz représente un ligand 1,2,4-triazole portant un substituant R sur l'azote en position 4 ;
- R est un groupe alkyle ou un groupe $R^1R^2N$- dans lequel $R^1$ et $R^2$ représentent chacun indépendamment de l'autre H ou un radical alkyle ;
- X représente au moins un anion monovalent ou divalent ;
- Y représente au moins un anion qui a un groupe chromophore ;
- b et c sont choisis de telle sorte que l'électroneutralité du composé (II) soit respectée. La couleur de chaque état de spin de ce matériau peut être ajustée en choisissant de manière appropriée le ou les anions X et Y, et en contrôlant leur proportions respectives dans le matériau.

Le document FR 2 755 696 décrit un composé de transition de spin incluant un réseau comprenant des molécules formées chacune d'un complexe métal-ligand et d'anion, dans lequel le métal est constitué par au moins un ion métallique ayant une configuration électronique en d4, d5, d6 oud d7, dans lequel le ligand comprend au moins un groupement aminotriazole, ou un groupement aminotriazole substitué, et dans lequel l'anion est formé d'un alliage d'au moins deux anions incluant un anion ayant un radical nitrate ($NO_3^-$). Ce document décrit également un dipositif d'affichage de données comprenant un système formant un écran et un système d'adressage thermique. Ce système d'adressage thermique comprend des moyens de chauffage B1 (comme un faisceau laser d'une longueur d'onde dans le domaine de l'infrarouge). Le faisceau laser a ainsi pour fonction, non pas de faire commuter le composé susmentionné, mais de suivre le phénomène de photocommutation.

Plusieurs méthodes permettant de provoquer la transition de spin de ces composés, c'est-à-dire permettant de faire passer ces composés d'un état BS à un HS ont déjà été proposées.

Selon EP-0 543 465 et FR 2 917 419, la transition de spin est provoquée par chauffage ou refroidissement et a lieu entre -20°C et +100°C. Le phénomène d'hystérésis peut aller de quelques degrés à quelques dizaines de degrés suivant les composés.

Certains auteurs tels que Freysz et al. (Chemical Physics Letters, 2004, 394, 318-323) proposent d'appliquer une radiation laser au centre de la boucle d'hystérésis thermique de matériaux à transition de spin à base de fer (complexe $[Fe(PM\text{-}BiA)_2(NCS)_2]$ avec PM-BiA = N-2'-pyridylméthylène-4-aminobiphényl) afin de provoquer une photoconversion de l'état BS à l'état HS. Selon ce document, l'irradiation est réalisée pendant quelques minutes à une longueur d'onde de 830 nm (proche infrarouge), à une puissance de 5 $mW.cm^{-2}$ et à une température de 10 K (soit -263,15°C environ). Au-delà d'une température de -100°C, la photocommutation selon ce procédé n'est plus observée.

D'autres auteurs, tels que Bonhommeau S. et al. (Angew. Chem. Int., 2005, 44, 4069) rapportent un essai d'irradiation laser unique d'un matériau à transition de spin de type $[Fe(pyrazine)\{Pt(CN)_4\}]$ à l'état déshydraté. Selon ce document, l'irradiation est réalisée dans le visible à une longueur d'onde de 532 nm avec des pulsations d'une durée de 8 ns. Cette technique présente cependant plusieurs inconvénients. Elle nécessite l'utilisation d'une impulsion laser nanoseconde ayant une énergie de plusieurs dizaines de millijoules. La longueur d'onde centrale de l'impulsion doit en outre être dans le domaine d'absorption du matériau.

Ces différentes méthodes d'irradiation laser utilisées jusqu'à ce jour sont basées sur l'effet LIESST (acronyme de l'expression anglophone « Light-Induced Excited Spin State Trapping ») qui est une photo-excitation électronique des ions métalliques, tels que les ions fer, des complexes à transition de spin.

L'inconvénient majeur de ces méthodes est qu'elles doivent être mises en oeuvre à des températures extrêmement basses (de l'ordre de -260°C environ) et qu'elles ne permettent pas la photocommutation des composés à transition de spin lorsqu'elles sont appliquées à température ambiante, ce qui est une limite importante pour l'application de ces procédés dans l'industrie.

La publication sicentifique de Gallé et al. ("Room temperature study of the optical switching of a spin crossover compound inside its thermal hysteresis loop", Applied Physics Letters, AIP, AMERICAN INSTITUTE OF PHYSCIS,

vol.96, no.4, du 27 janvier 2010, pages 41907-1 à 41907-3), décrit un procédé de photocommutation d'un matériau de transition de spin de l'état bas-spin vers l'état haut-spin comprenant une étape d'exposition dudit matériau à un rayonnement laser pulsé à une longueur d'onde comprise entre 0,355 μm et 0,532 μm, à température ambiante et à une puissance de 52 mJ.cm-2 pour 6 ns, soit 8,66.10+6 W/.cm-2. Ce procédé présente cependant l'inconvénient de nécessiter une puissance considérable et d'être par conséquent onéreux à mettre en oeuvre.

Les inventeurs se sont donc donné pour but de mettre au point un procédé qui puisse être mis en oeuvre de façon efficace à température ambiante pour faire passer les composés à transition de spin d'un état BS à un état HS.

Les inventeurs ont maintenant trouvé que, de manière surprenante, l'application d'un rayonnement laser à une longueur d'onde comprise dans l'infrarouge, dans certaines conditions, permet de faire commuter des matériaux à transition de spin d'un état BS à un état HS.

La présente invention a donc pour premier objet un procédé de photocommutation d'un matériau à transition de spin de l'état bas-spin vers l'état haut-spin comprenant un composé à base de fer(II) et d'un ligand triazole, caractérisé en ce qu'il comprend au moins une étape d'exposition dudit matériau à un rayonnement laser non polarisé à une longueur d'onde comprise dans l'infrarouge, à température ambiante et à une puissance de 1 $mW.cm^{-2}$ à 1 $W.cm^{-2}$.

Les inventeurs ont en effet établi que lorsqu'un matériau à transition de spin comprenant un composé à base de fer(II) et d'un ligand triazole est soumis à de telles conditions à température ambiante, on observe un accroissement localisé de la température du matériau photo-irradié générant sa commutation de l'état BS à l'état HS. Ce phénomène est totalement indépendant de l'effet LIESST et permet de provoquer la transition de spin à température ambiante alors que cela n'était jusqu'à présent pas envisageable en appliquant les méthodes de l'art antérieur.

Le procédé conforme à l'invention présente en outre les avantages suivants :

- il est applicable à tous les composés à transition de spin comprenant un composé à base de fer(II) et d'un ligand triazole,
- les puissances requises pour faire commuter optiquement les matériaux sont faibles et compatibles avec des micro sources lasers compacts et de faibles coûts,
- il est adapté à la photocommutation de tous types de composés à transition de spin comprenant un composé à base de fer (II) et d'un ligand triazole à l'état liquide ou solide,
- il permet de faire photo commuter les matériaux purs et/ou dilués dans différentes matrices,
- la zone de photocommutation peut facilement être contrôlée par la taille de la focalisation du rayonnement sur l'échantillon,
- le degré de photocommutation du composé est ajustable optiquement,
- la vitesse de balayage du composé que l'on désire faire photo commuter peut être contrôlée en fonction de la longueur d'onde et de la densité de puissance laser utilisée,
- la profondeur de l'échantillon sur laquelle la photocommutation est induite est ajustable : elle dépend de la puissance laser utilisée et de la vitesse de balayage,
- l'utilisation de rayonnements cohérents permet d'inscrire des motifs préprogrammés sans balayage de la surface de l'échantillon,
- la photocommutation est réversible ou irréversible à température ambiante en fonction de la nature du matériau à transition de spin sur lequel le procédé est appliqué,
- en ce qui concerne les matériaux dont la photocommutation est induite de manière irréversible, le procédé conduit à un adressage optique irréversible à température ambiante, particulièrement avantageux pour des applications de stockage de données, d'inscriptions en tout genre : publicité, marquage, dessin, art, décoration, détection, etc...
- il permet l'adressage optique de matériaux à transition de spin comprenant un composé à base de fer(II) et d'un ligand triazole.

Selon une forme de réalisation préférée du procédé conforme à l'invention, l'exposition dudit matériau est réalisée à une longueur d'onde variant de 0,78 à 30 μm, et encore plus préférentiellement de 5 à 20 μm environ.

Le rayonnement laser peut être continu ou impulsionnel.

Selon une forme de réalisation préférée, le matériau à transition de spin est soumis à un rayonnement laser continu.

La puissance du rayonnement laser varie de préférence de 80 à 500 $mW.cm^{-2}$, une puissance de l'ordre de 100 $mW.cm^{-2}$ étant tout particulièrement préférée.

La durée de l'exposition du matériau à transition de spin au rayonnement laser peut varier de quelques millisecondes à une puissance de 100 $mW.cm^{-2}$ à une centaine de secondes à une puissance de 1 $mW.cm^{-2}$.

La taille du rayonnement laser peut varier en fonction de la surface de l'échantillon que l'on souhaite faire photo commuter et de l'application que l'on souhaite donner au procédé. De façon générale, la taille du faisceau laser peut varier de 100 $\mu$m de diamètre à une puissance de 100 $mW.cm^{-2}$ à 1 mm à une puissance de 1 $W.cm^{-2}$.

Selon l'invention, le rayonnement laser peut être appliqué aussi bien à un matériau à transition de spin à l'état brut, c'est-à-dire sous la forme de particules, qu'à un matériau à transition de spin (MTS) incorporé sous forme dispersée dans une matrice solide, par exemple choisie parmi le plâtre, les vernis, les ciments, les peintures, et les matrices polymères (PPMA, PVA, etc...).

Les particules de MTS utilisables selon le procédé de l'invention sont de préférence de taille nanométrique ou micrométrique.

Par particules "nanométriques", on entend des particules qui ont un diamètre moyen entre 1 nm et 500 nm inclusivement, plus particulièrement entre 1 et 100 nm inclusivement.

Par particules "micrométriques", on entend des particules qui ont un diamètre moyen entre 1 $\mu$m et 500 $\mu$m inclusivement, et plus particulièrement entre 100 et 200 $\mu$m inclusivement.

Les matériaux à transition de spin utilisables selon le procédé conforme à la présente invention peuvent être choisis parmi les nombreux matériaux à transition de spin comprenant un composé à base de fer(II) et d'un ligand triazole décrits dans l'art antérieur et dont la transition de spin est réversible ou irréversible à température ambiante.

On peut notamment citer les MTS sous forme de particules nanométriques telles que décrites dans la demande internationale WO2007/065996. On peut citer en outre les matériaux à transition de spin sous forme de particules nanométriques ou micrométriques, qui présentent des couleurs variées avec une transition de spin définie, voire pour certains d'entres eux au moins deux températures de transition de spin, telles que décrites notamment dans la demande de brevet FR 2 917 410.

Les matériaux à transition de spin décrits dans la demande internationale WO2007/065996 et désignés ci-après par "matériaux (I)" sont constitués par des particules nanométriques comprenant essentiellement un composé répondant à la formule (I) suivante :

$$\left[\left(Fe_{1-y}M_yL_3\right)_w L_3\right]\left[X'_{2/x\left(1-z/x'\right)}\,Y'_{2z/x'}\right]_w \quad (I)$$

dans laquelle :

- L représente un ligand 1,2,4-triazole portant un substituant R sur l'azote en position 4 ;
- X' est un anion ayant la valence x, $1 \leq x \leq 2$ ;
- Y' est un anion différent de X ayant la valence x', $1 \leq x' \leq 2$
- R est un groupe alkyle ou un groupe $R^1R^2N$- dans lequel $R^1$ et $R^2$ représentent chacun indépendamment de l'autre H ou un radical alkyle ;
- M est un métal ayant une configuration $3d^4$, $3d^5$, $3d^6$ ou $3d^7$, autre que Fe ;
- $0 \leq y \leq 1$, $0 \leq z \leq 2$, et $3 \leq w \leq 1500$.

Lorsque w dans la formule (I) est respectivement 3, 300 ou 1500, la dimension moyenne des particules est respectivement d'environ 1 nm, 100 nm ou 500 nm.

Les matériaux à transition de spin décrits dans la demande de brevet FR 2 917 410, désignés ci-après par "matériaux (II)", sont constitués par au moins un composé répondant à la formule (II) suivante :

$$AX_bY_c \quad (II)$$

dans laquelle :

- A répond à la formule $Fe_{1-m}M_m(R\text{-}Trz)_3$ ;

- M est un métal ayant une configuration $3d^4$, $3d^5$, $3d^6$ ou $3d^7$, autre que Fe ;
- $0 \leq m \leq 1$ ;
- R-Trz représente un ligand 1,2,4-triazole portant un substituant R sur l'azote en position 4 ;
- R est un groupe alkyle ou un groupe $R^1R^2N$- dans lequel $R^1$ et $R^2$ représentent chacun indépendamment de l'autre H ou un radical alkyle ;
- X représente au moins un anion monovalent ou divalent ;
- Y représente au moins un anion qui a un groupe chromophore ;
- b et c sont choisis de telle sorte que l'électroneutralité du composé (II) soit respectée.

Dans la suite du texte, un ligand "1,2,4-triazole portant un substituant R sur l'azote en position 4" est désigné indifféremment par L ou par R-Trz.

Un matériau (II) peut être constitué par un seul composé (II) ayant un ou plusieurs anions colorants différents, ou par un mélange d'au moins deux composés qui répondent à la définition donnée ci-dessus pour (II).

Dans un composé (II), X peut représenter un ou plusieurs anions, de préférence un ou deux anions $X^1$ et $X^2$. Les anions sont choisis parmi les anions monovalents ou les anions divalents. Un anion monovalent peut être choisi parmi $BF_4^-$, $ClO_4^-$, $Br^-$ ; $Cl^-$, $NO_3^-$, $CF_3SO_3^-$, $CH_3SO_3^-$ et 3-nitrophénylsulfonate. Un anion divalent est choisi de préférence parmi $SO_4^{2-}$ et $CO_3^{2-}$. Comme exemples de mélange d'anions, on peut citer le couple $BF_4^-$ et $NO_3^-$, le couple $Br^-$ et $NO_3^-$, le couple $Cl^-$ et $NO_3^-$.

Y représente un ou plusieurs anions chromophores, de préférence un ou deux anions $Y^1$ et $Y^2$.

Un anion Y est choisi de préférence parmi les anions chromophores qui ont au moins deux cycles aromatiques et au moins un groupe $SO_3^-$. De tels anions sont fournis notamment par les composés dits "colorants acides" ou "colorants directs" ou "colorants brillants" ou "colorants à mordant".

L'anion Y de ces colorants peut comporter :

- un groupement azoïque, comme par exemple le C.I. Acid Orange 5 qui est le sel monosodique de l'acide 4-[[4-(phénylamino)-phényl]azo]benzènesulfonique, ou l'Acid Yellow 23 qui est la tartrazine, répondant à la formule :

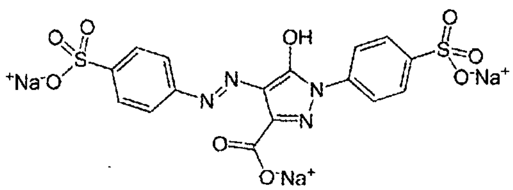

ou le Méthylorange ou Acid Orange 52 (hélianthine) répondant à la formule :

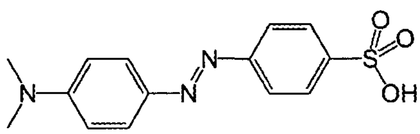

ou divers Direct Scarlet ; ou le C.I. Direct Blue 1, répondant à la formule :

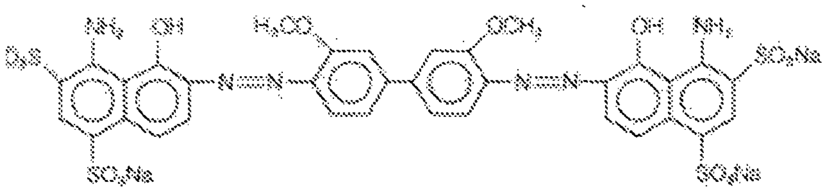

ou le C.I. Acid Red 27 répondant à la formule

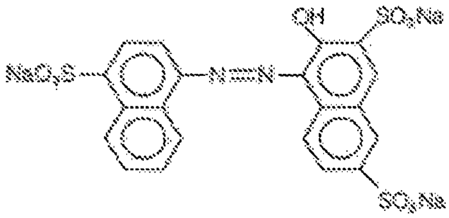

- un groupement dérivé d'une anthraquinone, par exemple le CI Reactive Blue 4 répondant à la formule :

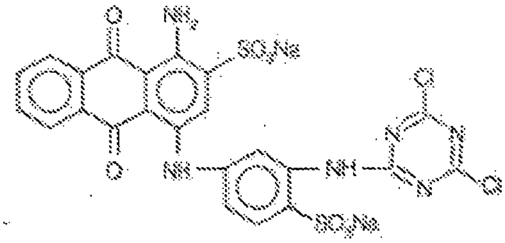

- un groupement dérivé d'une quinoline (C.I. Acid Yellow 3 ; D&C Yellow n° 30).

Dans un matériau (II), les proportions d'anions X et Y sont telles que la neutralité électronique du composé soit respectée.

La couleur de chaque état de spin d'un matériau (II) peut être ajustée en choisissant de manière appropriée le ou les anions X et Y, et en contrôlant leurs proportions respectives dans le matériau.

Il est nécessaire que la proportion d'anion colorant Y soit supérieure à un certain seuil afin d'éviter l'obtention d'un composé présentant une couleur rose à l'état bas spin et une couleur blanche à l'état haut spin.

Il est également nécessaire que la proportion d'anion colorant soit inférieure à un certain seuil au-delà duquel les couleurs correspondant à l'état bas spin et à l'état haut spin se distinguent difficilement.

Deux conditions sont à respecter :

1. un rapport molaire c/b (c'est-à-dire (nombre de mole de Y)/(nombre de mole de X) : il est inférieur ou égal à 0,1, et supérieur ou égal à $10^{-5}$ ;

2. une concentration limite en anion Y, comprise de manière générale entre $10^{-7}$ et $5.10^{-4}$ mole/L (par exemple pour Patent Blue entre $4.10^{-7}$ et $2.10^{-4}$ mole/L, et pour la tartrazine, entre $2{,}2.10^{-7}$ et $1{,}1.10^{-4}$ mol/L).

Parmi les MTS de formule (II) ci-dessus, ceux dans lesquels le substituant R du ligand 1,2,4-triazole est un radical hydroxyalkyle tel que par exemple un radical β-hydroxyéthyle, et X un anion 3-nitrophénylsulfonate, sont des MTS irréversibles, c'est-à-dire dont la transition de spin de l'état haut spin à l'état bas spin est irréversible à température ambiante après photocommutation selon le procédé conforme à l'invention. La photocommutation des MTS irréversibles s'accompagne d'un phénomène de désolvatation.

L'invention a en outre pour objet l'utilisation du procédé tel que défini ci-dessus pour le marquage de matériaux comprenant des particules d'au moins un composé à transition de spin comprenant un composé à base de fer(II) et d'un ligand triazole.

En fonction de la nature du composé à transition de spin, ledit marquage pourra être temporaire ou définitif. Ainsi lorsque le composé à transition de spin est un composé dit « réversible », c'est-à-dire dont le passage à l'état HS est réversible à température ambiante, alors le marquage peut être temporaire. Par contre, lorsque le composé à transition de spin est un composé dit « irréversible », c'est-à-dire qui reste à l'état HS à température ambiante, alors le marquage est permanent.

Le procédé conforme à l'invention trouve ainsi des applications dans tout type d'industrie, et notamment peut donc être utilisé dans le domaine du traitement de l'information, du stockage de données, de l'adressage optique, des sciences des matériaux de la plasturgie, des vernis, de la décoration, etc...

La présente invention est décrite plus en détails à l'aide des exemples qui suivent, qui sont donnés à titre d'illustration et auxquels l'invention n'est bien entendu pas limitée.

**EXEMPLES**

Les matières premières et matériels utilisés dans les exemples qui suivent sont listées ci-après :

- [Fe(R-trz)$_3$NO$_3$] avec R = NH$_2$ et dont la préparation est décrite dans la demande de brevet FR 2 917 410 ;
- [Fe(R-trz)$_3$X$_2$] avec R = -(CH$_2$)$_2$OH et X = 3-nitrophényl-sulfonate, et dont la préparation est décrite dans la demande de brevet FR 2 917 410
- Vernis incolore pour métaux vendu sous la dénomination commerciale Vernis Métaux, le Secret de l'Ebéniste ® par la société Syntilor ;
- Plâtre de bâtiment, vendu sous la dénomination commerciale Enduit de Rebouchage Poudre Chrono, par la société Toupret ;
- Polyméthylméthacrylate (PMMA) vendu sous la référence M.W. 38000 Beads par la société Acros Organics ;
- Peinture Laque Glycéro blanche vendue sous la référence Base P, Gamme Prestige par la société Tollens ;
- Acétonitrile pur à 99 %(Aldrich)
- Laser $CO_2$, modèle Duo Lase®, vendu par la société Synrad Inc..

Les composés à transition de spin ont été placés devant une source laser délivrant en continu une centaine de milliwatts dans l'infrarouge. Le rayonnement non polarisé émis par cette source a été utilisé directement sur le matériau, sans précaution particulière. Pour enregistrer la photocommutation, l'échantillon a été déplacé devant le faisceau laser soit à la main par l'expérimentateur, soit en utilisant une table micrométrique permettant de contrôler la vitesse de balayage.

**Exemple 1**

<u>Photocommutation d'un composé à transition de spin de la famille des Fer(II) R-triazole</u>

Dans cet exemple on a réalisé la photocommutation réversible du composé suivant : [Fe(NH$_2$trz)$_3$NO$_3$]
L'échantillon de [Fe(NH$_2$trz)$_3$NO$_3$], sous forme d'une poudre de grains micrométriques, a été placé sous le rayonnement laser à une puissance de 150 mW.cm$^{-2}$. La longueur d'onde du laser était de 10,6 $\mu$m. Le composé n'a été exposé que pendant quelques millisecondes.
La figure 1 annexée est une photographie du matériau avant l'irradiation (1a), pendant l'irradiation laser (1b) et après l'irradiation (1c).
Après photo-excitation ces composés, roses dans l'état BS, deviennent blancs dans l'état HS. On observe que le composé a été complètement photo commuté.

**Exemple 2**

<u>Photocommutation d'un composé à transition de spin de la famille des Fer(II) R-triazole inséré dans une matrice polymère</u>

Dans cet exemple on a réalisé la photocommutation réversible du [Fe(NH$_2$trz)$_3$NO$_3$] après l'avoir inséré dans une matrice polymère constituée d'un vernis incolore pour métaux vendu sous la dénomination commerciale Vernis Métaux, le Secret de l'Ebéniste ® par la société Syntilor, à raison de 5 % en masse.
L'échantillon a été placé sur une table à translation pilotée par ordinateur. Un interrupteur, lui aussi piloté par ordinateur, permettait d'interrompre l'illumination laser de l'échantillon. La vitesse de balayage, ajustable par ordinateur, a été réglée à 1 cm.s$^{-1}$, et la puissance du laser était de 100 mW.cm$^{-2}$. Le diamètre du faisceau laser était de 1 mm. L'irradiation a été faite à une longueur d'onde de 10,6 $\mu$m.
Dans cet exemple, des lettres ont été inscrites sur l'échantillon, chaque lettre a été inscrite par un seul balayage laser, le faisceau laser étant bloqué avant l'inscription de chacune des lettres.
La figure 2 annexée est une photographie de la matrice incorporant le matériau à transition avant irradiation (2a) et après irradiation (2b). Dans ce cas l'irradiation et le balayage de l'échantillon ont été réalisés de façon à obtenir le graphisme « ICMCB » à la surface de la matrice.

**Exemple 3**

Photocommutation d'un composé à transition de spin de la famille des Fer(II) R-triazole inséré dans une matrice d'enduit de rebouchage (plâtre)

Dans cet exemple on a réalisé la photocommutation réversible du $[Fe(NH_2\text{-trz})_3NO_3]$, après l'avoir inséré, à raison de 5 % massique, dans une matrice d'enduit de rebouchage (plâtre) constituée d'1 g de plâtre en poudre pour 10 ml d'éthanol.

Le même système d'inscription que celui utilisé ci-dessus à l'exemple 2 a également été utilisé dans cet exemple. Le diamètre du faisceau laser sur l'échantillon était de 2 mm. La vitesse de balayage a été réglée à 1 $cm.s^{-1}$ et la puissance du laser à 100 $mW.cm^{-2}$. L'irradiation a été faite à une longueur d'onde de 10,6 $\mu$m. Chaque lettre a été écrite par un seul balayage laser, le faisceau étant bloqué avant l'inscription de chacune des lettres.

La figure 3 annexée est une photographie de la matrice incorporant le matériau à transition de spin avant irradiation (3a) et après irradiation (3b), dans ce cas l'irradiation et le balayage de l'échantillon ont été réalisés de façon à obtenir le graphisme « CPMOH » à la surface de la matrice.

**Exemple 4**

Photocommutation d'un composé à transition de spin « irréversible »de la famille des Fer(II) R-triazole

Dans cet exemple, on a réalisé la photocommutation du composé $[Fe(R\text{-trz})_3X_2]$ avec R =$-(CH_2)_2OH$ et X = 3-nitrophényl-sulfonate, à l'état de poudre pure ou après son insertion dans une matrice polymère (PMMA) ou dans une peinture.

Incorporation dans la matrice polymère : Le composé à transition de spin a été incorporé à raison de 5 % massique dans la matrice polymère préalablement préparée par dissolution de 1,2 g de PMMA dans l'acétonitrile à 40°C. Après dépôt sur une plaque de plexiglas et séchage, on a obtenu un matériau constitué d'une matrice de PMMA dans laquelle le composé à transition de spin était dispersé.

Incorporation dans la peinture : Le composé à transition de spin a été incorporé à raison de 10 % massique dans une peinture laque glycéro. Après dépôt sur une plaque vendue sous la dénomination commerciale Opacity Charts par la société Leneta et séchage, on a obtenu un matériau constitué d'une peinture dans laquelle le composé à transition de spin était dispersé.

Chacun des échantillons a été éclairé par un faisceau laser d'un diamètre de 1 mm à une puissance de 150 $mW.cm^{-2}$. L'irradiation a été faite à une longueur d'onde de 10,6 $\mu$m.

La figure 4 annexée montre des photographies de chacun des matériaux prises avant et après irradiation, sur le matériau brut, c'est-à-dire à sur le composé à transition de spin l'état de poudre (4a), sur le matériau constitué par la matrice de PMMA incorporant le composé à transition de spin (4b) et sur le matériau constitué par la peinture incorporant le matériau à transition de spin (4c).

**Exemple 5**

Photocommutation d'un composé à transition de spin irréversible de la famille des Fer(II) R-triazole inséré dans une matrice polymère

Dans cet exemple on a réalisé la photocommutation irréversible du composé $[Fe(R\text{-trz})_3X_2]$ avec R =$-(CH_2)_2OH$ et X = 3-nitrophényl-sulfonate.

Le composé étudié était sous forme d'une poudre de grains micrométrique. L'échantillon a été éclairé par un faisceau laser ayant un diamètre d'environ 1 mm et dont la puissance était de 150 $mW.cm^{-2}$. L'échantillon a été déplacé, à la main, par le manipulateur.

La figure 5 annexée montre l'évolution de la susceptibilité magnétique et de la couleur de la poudre en fonction de la température de l'échantillon.

Un simple procédé de traitement thermique (procédé ne faisant pas partie de la présente invention) permet à la poudre initialement rose dans l'état bas spin de devenir blanche à haute température. Après un seul cycle de chauffage, l'échantillon n'a recouvré sa couleur initiale (rose) qu'à très basse température. ($T<100$ K). Le changement de l'état de spin du composé a été confirmé par les mesures de susceptibilité magnétique présentées.

Ce même composé n'ayant subit aucun traitement thermique préalable (Etat BS - figure 5b) a été exposé à un faisceau laser selon le procédé conforme à la présente invention. La puissance et la longueur d'onde du rayonnement étaient de 150 $mW.cm^{-2}$ et 10,6 $\mu$m respectivement. La zone éclairée est devenue blanche (figure 5a). La température de l'échantillon a alors été baissée en dessous de 100 K, l'ensemble de l'échantillon est redevenu rose. La partie ayant

été exposée au rayonnement laser a repris sa couleur blanche dès que la température de l'échantillon a été ramenée au-dessus de 130 K et les zones éclairées et non éclairées par le faisceau laser apparaissent clairement (figure 5c). Enfin, l'échantillon a été chauffé au-dessus de 360 K, l'ensemble du composé est alors devenu blanc. Le comportement en température du composé est alors similaire à celui d'un échantillon chauffé.

## Revendications

1. Procédé de photocommutation d'un matériau à transition de spin de l'état bas-spin vers l'état haut-spin comprenant un composé à base de fer(II) et de ligand triazole, **caractérisé en ce qu'** il comprend au moins une étape d'exposition dudit matériau à un rayonnement laser non polarisé à une longueur d'onde comprise dans l'infrarouge, à température ambiante et à une puissance de 1 $mW.cm^{-2}$ à 1 $W.cm^{-2}$.

2. Procédé selon la revendication 1, **caractérisé en ce que** l'exposition dudit matériau est réalisée à une longueur d'onde variant de 0,78 à 30 $\mu$m.

3. Procédé selon la revendication 1 ou 2, **caractérisé en ce que** le matériau à transition de spin est soumis à un rayonnement laser continu.

4. Procédé selon l'une quelconque des revendications précédentes, **caractérisé en ce que** la puissance du rayonnement laser varie de 80 à 500 $mW.cm^{-2}$.

5. Procédé selon l'une quelconque des revendications précédentes, **caractérisé en ce que** la durée de l'exposition du matériau à transition de spin au rayonnement laser varie de quelques millisecondes à une puissance de 100 $mW.cm^{-2}$ à une centaine de secondes à une puissance de 1 $mW.cm^{-2}$.

6. Procédé selon l'une quelconque des revendications précédentes, **caractérisé en ce que** la taille du faisceau laser varie de 100 $\mu$m de diamètre à une puissance de 100 $mW.cm^{-2}$ à 1 mm à une puissance de 1 $W.cm^{-2}$.

7. Procédé selon l'une quelconque des revendications précédentes, **caractérisé en ce que** le matériau à transition de spin est sous forme de particules ou incorporé, sous forme dispersé, dans une matrice solide.

8. Procédé selon la revendication 7, **caractérisé en ce que** la matrice solide est choisie parmi le plâtre, les vernis, les ciments, les peintures, et les matrices polymères.

9. Procédé selon l'une quelconque des revendications précédentes, **caractérisé en ce que** le matériau à transition de spin est choisi parmi les particules nanométriques comprenant essentiellement un composé répondant à la formule (I) suivante :

$$\left[\left(Fe_{1-y}M_yL_3\right)_w L_3\right]\left[X'_{2/x\left(1-z/x'\right)}\,Y'_{2z/x'}\right]_w \quad \text{(I)}$$

dans laquelle :

- L représente un ligand 1,2,4-triazole portant un substituant R sur l'azote en position 4 ;
- X' est un anion ayant la valence x, $1 \leq x \leq 2$ ;
- Y' est un anion différent de X ayant la valence x', $1 \leq x' \leq 2$ ;
- R est un groupe alkyle ou un groupe $R^1R^2N$- dans lequel $R^1$ et $R^2$ représentent chacun indépendamment de l'autre H ou un radical alkyle ;
- M est un métal ayant une configuration $3d^4$, $3d^5$, $3d^6$ ou $3d^7$, autre que Fe ;
- $0 \leq y \leq 1$, $0 \leq z \leq 2$, et $3 \leq w \leq 1500$.

10. Procédé selon l'une quelconque des revendications 1 à 8, **caractérisé en ce que** le matériau à transition de spin est choisi parmi les matériaux constitués par au moins un composé répondant à la formule (II) suivante :

$AX_bY_c$ (II)

dans laquelle :

- A répond à la formule $Fe_{1-m}M_m(R\text{-}Trz)_3$ ;
- M est un métal ayant une configuration $3d^4$, $3d^5$, $3d^6$ ou $3d^7$, autre que Fe ;
- $0 \leq m \leq 1$ ;
- R-Trz représente un ligand 1,2,4-triazole portant un substituant R sur l'azote en position 4 ;
- R est un groupe alkyle ou un groupe $R^1R^2N$- dans lequel $R^1$ et $R^2$ représentent chacun indépendamment de l'autre H ou un radical alkyle ;
- X représente au moins un anion monovalent ou divalent ;
- Y représente au moins un anion qui a un groupe chromophore ;
- b et c sont choisis de telle sorte que l'électroneutralité du composé (II) soit respectée.

**11.** Procédé selon la revendication 10, **caractérisé en ce que** Y est choisi parmi les anions chromophores qui ont au moins deux cycles aromatiques et au moins un groupe $SO_3^-$.

**12.** Procédé selon la revendication 10, **caractérisé en ce que** le matériau à transition de spin est un matériau dont la transition de spin de l'état haut spin à l'état bas spin est irréversible à température ambiante et est choisi parmi les composés de formule (II) dans lesquels le substituant R du ligand 1,2,4-triazole est un radical hydroxyalkyle et X un anion 3-nitrophénylsulfonate.

**13.** Utilisation du procédé tel que défini à l'une quelconque des revendications précédentes, pour le marquage de matériaux comprenant des particules d'au moins un composé à transition de spin comprenant un composé à base de fer(II) et d'un ligand triazole.

**14.** Utilisation du procédé tel que défini à l'une quelconque des revendications 1 à 12, pour le traitement de l'information, le stockage de données ou l'adressage optique.

**Patentansprüche**

**1.** Verfahren für die Photoschaltung eines Spin-Übertragungsmaterials aus dem Low-Spin-Zustand in den High-Spin-Zustand, das eine Verbindung auf der Basis von Eisen(II) und von Triazol-Ligand umfasst, **dadurch gekennzeichnet, dass** es mindestens einen Schritt der Exposition des Materials gegenüber einer nicht polarisierten Laserstrahlung mit einer Wellenlänge inklusive im Infrarotbereich bei Raumtemperatur und mit einer Leistung von 1 $mW.cm^{-2}$ bis 1 $W.cm^{-2}$ umfasst.

**2.** Verfahren nach Anspruch 1, **dadurch gekennzeichnet, dass** die Exposition des Materials mit einer Wellenlänge durchgeführt wird, die von 0,78 bis 30 $\mu$m schwankt.

**3.** Verfahren nach Anspruch 1 oder 2, **dadurch gekennzeichnet, dass** das Spin-Übertragungsmaterial einer kontinuierlichen Laserstrahlung ausgesetzt wird.

**4.** Verfahren nach einem der vorangehenden Ansprüche, **dadurch gekennzeichnet, dass** die Leistung der Laserstrahlung von 80 bis 500 $mW.cm^{-2}$ schwankt.

**5.** Verfahren nach einem der vorangehenden Ansprüche, **dadurch gekennzeichnet, dass** die Dauer der Exposition des Spin-Übertragungsmaterials gegenüber der Laserstrahlung von einigen Millisekunden mit einer Leistung von 100 $mW.cm^{-2}$ bis zirka einhundert Sekunden mit einer Leistung von 1 $mW.cm^{-2}$ schwankt.

**6.** Verfahren nach einem der vorangehenden Ansprüche, **dadurch gekennzeichnet, dass** die Größe des Laserstrahl von 100 $\mu$m Durchmesser mit einer Leistung von 100 $mW.cm^{-2}$ bis 1 mm mit einer Leistung von 1 $W.cm^{-2}$ schwankt

**7.** Verfahren nach einem der vorangehenden Ansprüche, **dadurch gekennzeichnet, dass** das Spin-Übertragungsmaterial in Partikelform oder in verteilter Form in eine feste Matrix inkorporiert ist.

**8.** Verfahren nach Anspruch 7, **dadurch gekennzeichnet, dass** die feste Matrix aus dem Gips, den Lacken, den

Zementen, den Farben und den Polymermatrizes ausgewählt ist.

**9.** Verfahren nach einem der vorangehenden Ansprüche, **dadurch gekennzeichnet, dass** das Spin-Übertragungsmaterial aus den Nanometerpartikeln ausgewählt ist, die im Wesentlichen eine Verbindung umfassen, die der folgenden Formel (I) entspricht:

$$\left[\left(Fe_{1-y}M_{y}L_{3}\right)_{w}L_{3}\right]\left[X'_{2/x\left(1-z/x'\right)}\,Y'_{2z/x'}\right]_{w} \quad \text{(I)}$$

wobei:

- L einen 1,2,4-Triazol-Liganden mit einem Substituenten R auf dem Stickstoff in Position 4 darstellt,
- X’ ein Anion mit der Wertigkeit x, $1 \leq x \leq 2$, ist,
- Y’ ein von X unterschiedliches Anion mit der Wertigkeit x’, $1 \leq x' \leq 2$, ist,
- R eine Alkylgruppe oder eine Gruppe $R^1R^2N$- ist, in der $R^1$ und $R^2$ jeweils unabhängig von dem anderen H oder ein Alkylradikal darstellen,
- M ein Metall ist mit einer Konfiguration $3d^4$, $3d^5$, $3d^6$ oder $3d^7$, anders als Fe,
- $0 \leq y \leq 1$, $0 \leq z \leq 2$ und $3 \leq w \leq 1500$.

**10.** Verfahren nach einem der Ansprüche 1 bis 8, **dadurch gekennzeichnet, dass** das Spin-Übertragungsmaterial aus den Materialien ausgewählt ist, die von mindestens einer Verbindung gebildet werden, die der folgenden Formel (II) entspricht:

$$AX_bY_c \quad \text{(II)}$$

wobei:

- A der Formel $Fe_{1-m}M_m(R\text{-}Trz)_3$ entpricht,
- M ein Metall ist mit einer Konfiguration $3d^4$, $3d^5$, $3d^6$ oder $3d^7$, anders als Fe,
- $0 \leq m \leq 1$,
- R-Trz einen 1,2,4-Triazol-Liganden mit einem Substituenten R auf dem Stickstoff in Position 4 darstellt,
- R eine Alkylgruppe oder eine Gruppe $R^1R^2N$- ist, in der $R^1$ und $R^2$ jeweils unabhängig von dem anderen H oder ein Alkylradikal darstellen,
- X mindestens ein einwertiges oder zweiwertiges Anion darstellt,
- Y mindestens ein Anion darstellt, das eine chromophore Gruppe hat,
- b und c derart ausgewählt sind, dass die Elektroneutralität der Verbindung (II) respektiert ist.

**11.** Verfahren nach Anspruch 10, **dadurch gekennzeichnet, dass** Y aus den chromophoren Anionen ausgewählt ist, die mindestens zwei aromatische Zyklen und mindestens eine $SO_3^-$-Gruppe haben.

**12.** Verfahren nach Anspruch 10, **dadurch gekennzeichnet, dass** das Spin-Übertragungsmaterial ein Material ist, dessen Spin-Übertragung aus dem Low-Spin-Zustand in den High-Spin-Zustand bei Raumtemperatur irreversibel ist und aus den Verbindungen der Formel (II) ausgewählt ist, wobei der Substituent R des 1,2,4-Triazol-Liganden ein Hydroxyalkylradikal und X ein 3-Nitrophenylsulfonatanion ist.

**13.** Verwendung des Verfahrens nach einem der vorangehenden Ansprüche zur Markierung von Materialien, die Partikel mindestens eines Spin-Übertragungsmaterials mit einer Verbindung auf der Basis von Eisen(II) und eines Triazol-Liganden umfassen.

**14.** Verwendung des Verfahrens nach einem der Ansprüche 1 bis 12 zur Informationsverarbeitung, Datenspeicherung oder optischen Adressierung.

## Claims

1. A process for the photoswitching of a spin-transition material from the low spin state to the high spin state comprising a compound based on iron(II) and on a triazole ligand, **characterized in that** it comprises at least one stage of exposure of said material to unpolarized laser radiation at a wavelength within the infrared range, at ambient temperature and at a power of from 1 $mW.cm^{-2}$ to 1 $W.cm^{-2}$.

2. The process as claimed in claim 1, **characterized in that** the exposure of said material is carried out at a wavelength varying from 0.78 to 30 $\mu$m.

3. The process as claimed in claim 1 or 2, **characterized in that** the spin-transition material is subjected to continuous laser radiation.

4. The process as claimed in any one of the preceding claims, **characterized in that** the power of the laser radiation varies from 80 to 500 $mW.cm^{-2}$.

5. The process as claimed in any one of the preceding claims, **characterized in that** the duration of the exposure of the spin-transition material to the laser radiation varies from a few milliseconds at a power of 100 $mW.cm^{-2}$ to approximately a hundred seconds at a power of 1 $mW.cm^{-2}$.

6. The process as claimed in any one of the preceding claims, **characterized in that** the size of the laser beam varies from 100 $\mu$m in diameter at a power of 100 $mW.cm^{-2}$ to 1 mm at a power of 1 $W.cm^{-2}$.

7. The process as claimed in any one of the preceding claims, **characterized in that** the spin-transition material is in the form of particles or is incorporated in dispersed form in a solid matrix.

8. The process as claimed in claim 7, **characterized in that** the solid matrix is chosen from plaster, varnishes, cements, paints and polymer matrices.

9. The process as claimed in any one of the preceding claims, **characterized in that** the spin-transition material is chosen from nanometric particles essentially comprising a compound corresponding to the following formula (I):

$$\left[\left(Fe_{1-y}M_yL_3\right)_wL_3\right]\left[X'_{2/x\left(1-z/x'\right)}Y'_{2z/x'}\right]_w \quad (I)$$

in which:

- L represents a 1,2,4-triazole ligand carrying an R substituent on the nitrogen in the 4 position;
- X' is an anion having the valency x, $I \leq x \leq 2$;
- Y' is an anion other than X having the valency x', $1 \leq x' \leq 2$;
- R is an alkyl group or an $R^1R^2N$- group in which $R^1$ and $R^2$ each represent, independently of the other, H or an alkyl radical;
- M is a metal having a $3d^4$, $3d^5$, $3d^6$ or $3d^7$ configuration, other than Fe;
- $0 \leq y \leq 1$, $0 \leq z \leq 2$ and $3 \leq w \leq 1500$.

10. The process as claimed in any one of claims 1 to 8, **characterized in that** the spin-transition material is chosen from materials composed of at least one compound corresponding to the following formula (II):

$$AX_bY_c \quad (II)$$

in which:

- A corresponds to the formula $Fe_{1-m}M_m(R\text{-}Trz)_3$;
- M is a metal having a $3d^4$, $3d^5$, $3d^6$ or $3d^7$ configuration, other than Fe;
- $0 \leq m \leq 1$;

- R-Trz represents a 1,2,4-triazole ligand carrying an R substituent on the nitrogen in the 4 position;
- R is an alkyl group or a group $R^1R^2N$- in which $R^1$ and $R^2$ each represent, independently of the other, H or an alkyl radical;
- X represents at least one monovalent or divalent anion;
- Y represents at least one anion which has a chromophore group;
- b and c are chosen so that the electrical neutrality of the compound (II) is respected.

**11.** The process as claimed in claim 10, **characterized in that** Y is chosen from chromophoric anions which have at least two aromatic rings and at least one $SO_3^-$ group.

**12.** The process as claimed in claim 10, **characterized in that** the spin-transition material is a material for which the spin transition from the high spin state to the low spin state is irreversible at ambient temperature and which is chosen from the compounds of formula (II) in which the R substituent of the 1,2,4-triazole ligand is a hydroxyalkyl radical and X is a 3-nitrophenylsulfonate anion.

**13.** The use of the process as defined in any one of the preceding claims for the marking of materials comprising particles of at least one spin-transition compound comprising a compound based on iron(II) and on a triazole ligand.

**14.** The use of the process as defined in any one of claims 1 to 12 in information processing, data storage or optical addressing.

**Fig. 1**

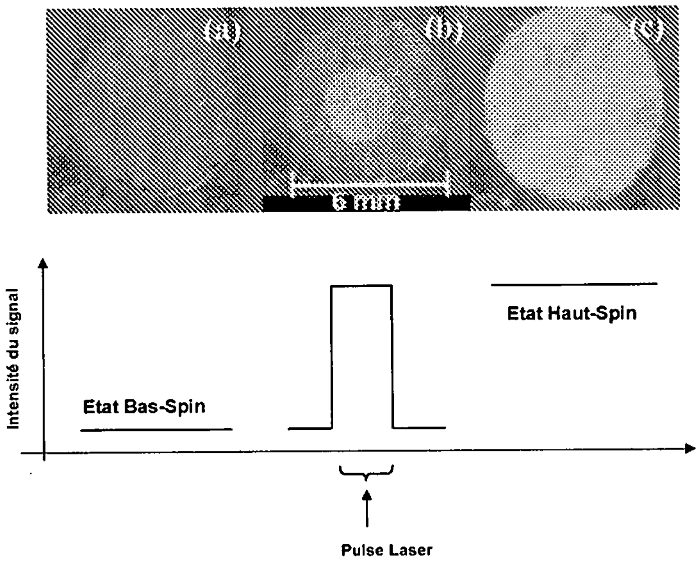


**Fig. 2**

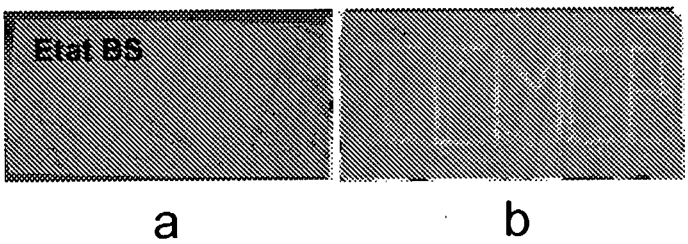


**Fig. 3**

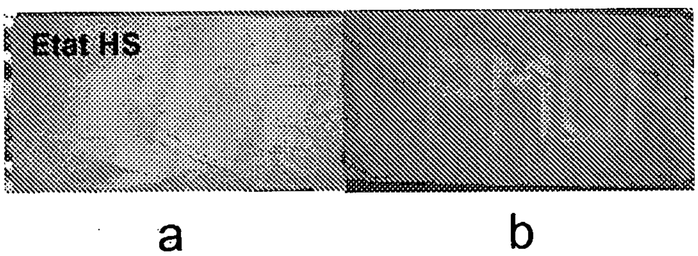

Fig. 4

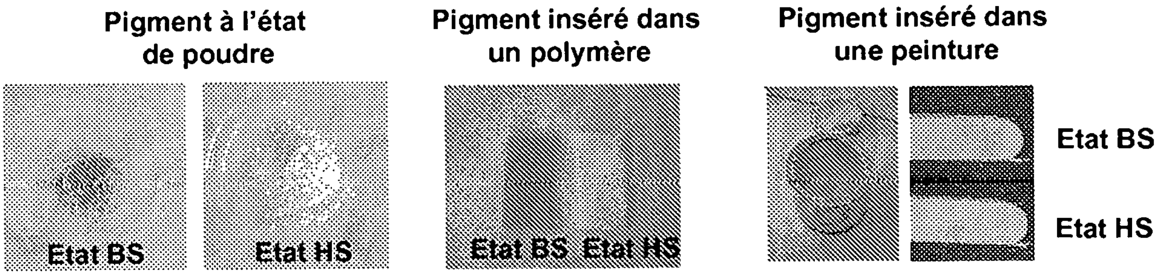
Pigment à l'état de poudre
Pigment inséré dans un polymère
Pigment inséré dans une peinture
Etat BS
Etat HS
Etat BS
Etat HS
Etat BS
Etat HS

Fig. 5

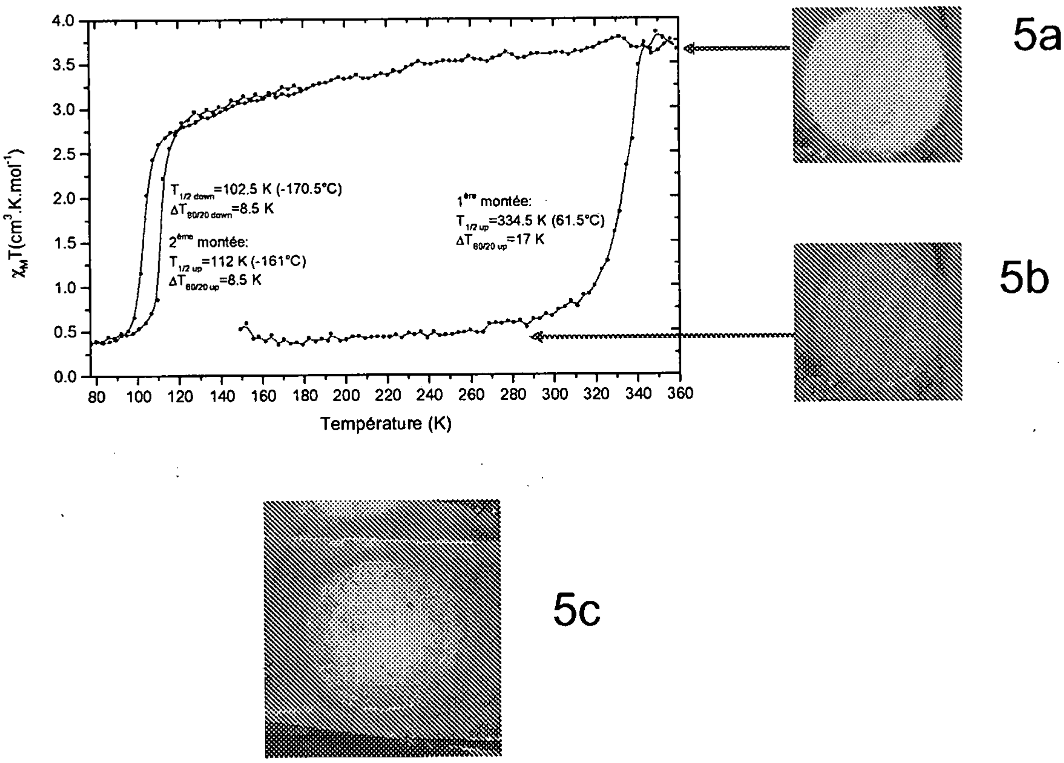
χMT(cm3.K.mol-1)
Température (K)
T1/2 down=102.5 K (-170.5°C)
ΔT80/20 down=8.5 K
2ème montée:
T1/2 up=112 K (-161°C)
ΔT80/20 up=8.5 K
1ère montée:
T1/2 up=334.5 K (61.5°C)
ΔT80/20 up=17 K
5a
5b
5c

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 0543465 A
- EP 0666561 A
- EP 0745986 A
- EP 0842988 A
- WO 2007065996 A
- FR 2917410
- FR 2755696
- FR 2917419

### Littérature non-brevet citée dans la description

- **FREYSZ et al.** *Chemical Physics Letters,* 2004, vol. 394, 318-323
- **BONHOMMEAU S. et al.** *Angew. Chem. Int.,* 2005, vol. 44, 4069
- Room temperature study of the optical switching of a spin crossover compound inside its thermal hysteresis loop. **GALLÉ et al.** Applied Physics Letters. AIP, AMERICAN INSTITUTE OF PHYSCIS, 27 Janvier 2010, vol. 96, 41907-141907-3